# EUROPÄISCHE PATENTSCHRIFT

(11) **EP 1 971 568 B1**
(45) Veröffentlichungstag und Bekanntmachung des Hinweises auf die Patenterteilung: **03.08.2011**
(21) Anmeldenummer: 06841544.7
(22) Anmeldetag: 21.12.2006
(51) Int. Cl.: C07C 209/18, C07C 211/45

(54) **VERFAHREN ZUR KONTINUIERLICHEN HERSTELLUNG EINES PRIMÄREN AROMATISCHEN AMINS**
PROCESS FOR CONTINUOUS PREPARATION OF A PRIMARY AROMATIC AMINE
PROCEDE DE FABRICATION EN CONTINU D'UNE AMINE AROMATIQUE PRIMAIRE

(30) Priorität: 05.01.2006 DE 102006000995
(43) Veröffentlichungstag der Anmeldung: 24.09.2008
(73) Patentinhaber: BASF SE, 67056 Ludwigshafen (DE)
(72) Erfinder: HAESE, Frank, 63128 Dietzenbach (DE); WULFF-DÖRING, Joachim, 67227 Frankenthal (DE); KÖHLER, Ulrich, 68259 Mannheim (DE); GAA, Peter, 67551 Worms (DE); PAPE, Frank-Friedrich, 67259 Kleinniedesheim (DE); MELDER, Johann-Peter, 67459 Böhl-Iggelheim (DE); JULIUS, Manfred, 67117 Limburgerhof (DE)
(86) Internationale Anmeldenummer: PCT/EP2006/070074
(87) Internationale Veröffentlichungsnummer: WO 2007/077148

(56) Entgegenhaltungen:
- EP-A1- 0 050 229
- EP-A2- 0 701 995
- DATABASE CA [Online] CHEMICAL ABSTRACTS SERVICE, COLUMBUS, OHIO, US; NOEL, MICHAEL ET AL: "Electrochemical preparation of 2-amino-m-xylene from 2-nitro-m-xylene" XP002425457 gefunden im STN Database accession no. 1982:112280 & IN 148 694 A1 (COUNCIL OF SCIENTIFIC AND INDUSTRIAL RESEARCH, INDIA) 16. Mai 1981 (1981-05-16)

## Beschreibung

Die vorliegende Erfindung betrifft ein Verfahren zur kontinuierlichen Herstellung eines primären aromatischen Amins durch Umsetzung eines entsprechenden cycloaliphatischen Alkohols mit Ammoniak in Gegenwart von Wasserstoff bei einer Temperatur im Bereich von 80 bis 350°C in Gegenwart eines Heterogenkatalysators.

Aromatische Amine sind wichtige Ausgangsverbindungen für die Herstellung von Medikamenten und Pflanzenschutzwirkstoffen. Weiterhin finden sie Verwendung bei der Herstellung von Kunststoffen.
Diese Verbindungen sind daher von großer wirtschaftlicher Bedeutung und für ihre Herstellung wurden verschiedene Verfahren entwickelt.

Eine bekannte Methode ist die Hydrierung von aromatischen Nitroverbindungen (Houben-Weyl, Methoden der organischen Chemie, 4. Auflage Band 11/1, Seite 360 ff). Nachteilig ist, dass die vorausgehende Nitrierung bei substituierten Aromaten oft ein Gemisch mehrerer Nitro-Produkte oder Regio-Isomere liefert.

Um ein einheitliches Produkt zu erhalten, besteht die Möglichkeit, Anilin und substituierte Aniline aus den entsprechenden Cyclohexanol- und/oder Cyclohexanon-Derivaten zu synthetisieren.

US 3,553,268 (Witco Chemical Comp.) lehrt die Herstellung von Anilin aus Gemischen von Cyclohexanol, Cyclohexanon und Ammoniak an einem Nickelkatalysator. Als nachteilig erscheint hier, dass dieses Verfahren nicht auf substituierte aromatische Amine anwendbar ist. Zudem wird es dadurch nachteilig eingeschränkt, dass offenbar nur Gemische enthaltend maximal 65 Gew.-% Cyclohexanol eingesetzt werden können.

US 3,442,950 und US 3,347,921 (beide Halcon International, Inc.) lehren die Umsetzung von Gemischen aus Cyclohexanol und Cyclohexanon und Ammoniak oder primären Aminen an Dehydrierkatalysatoren, z.B. Pt/C. Nachteilig sind hier die Bildung höhersiedender Produkte und anderer Nebenkomponenten, wie Imine, Cyclohexylamin, Phenol, Diphenylamin und Phenylcyclohexylamin, was eine aufwendige Verfahrensführung und Aufarbeitung notwendig macht.

EP 50 229 A1 (BASF AG) beschreibt die Umsetzung von Cyclohexanol, Cyclohexanol-Derivaten, Cyclohexanon, Cyclohexanon-Derivaten oder Mischungen davon in einer Ammoniak/Wasserstoffatmosphäre und an einem Palladiumkatalysator. Nachteilig an diesen Verfahren ist, dass zur Erreichung guter Ausbeuten der Katalysator neben Palladium noch Zink und das sehr giftige Cadmium enthalten muss. Werden Zink und Cadmium weggelassen, verschlechtern sich die Ausbeuten erheblich. Wird an dem Katalysator das Cadmium weggelassen, so müssen augenscheinlich für eine gute Ausbeute diverse aliphatische Amine, wie N-Methylmorpholin oder N-Methylpiperidin, zugesetzt werden. In weiteren Beispielen können an dem Katalysator zwar auch ohne Cadmium und auch ohne zugesetztem aliphatischen Amin die entsprechenden aromatischen Amine in guten Ausbeuten synthetisiert werden. Dies gelingt jedoch nur, weil die Menge an Palladium im Katalysator entweder drastisch erhöht oder die Formkörper stark verkleinert werden. Nachteilig ist damit, dass ein hoher Edelmetallgehalt den Katalysator sehr teuer macht und zu kleine Formkörper den Anstaudruck ungünstig erhöhen und problematisch in der Handhabung sind.

Der vorliegenden Erfindung lag die Aufgabe zugrunde, unter Überwindung eines Nachteils oder mehrerer Nachteile des Stands der Technik, ein verbessertes wirtschaftliches Verfahren zur Herstellung eines primären aromatischen Amins aufzufinden. Ziel war es insbesondere, ein Verfahren zu finden, nach welchem aus Cyclohexanol und substituierten Cyclohexanolen Anilin und entsprechend substituierte aromatische Amine in guten Ausbeuten herstellbar sind, ohne die Nachteile des Stands der Technik, insbesondere die oben beschriebenen Nachteile, in Kauf nehmen zu müssen.

Demgemäß wurde ein Verfahren zur kontinuierlichen Herstellung eines primären aromatischen Amins durch Umsetzung eines entsprechenden cycloaliphatischen Alkohols mit Ammoniak in Gegenwart von Wasserstoff bei einer Temperatur im Bereich von 80 bis 350°C in Gegenwart eines Heterogenkatalysators gefunden, welches dadurch gekennzeichnet ist, dass die katalytisch aktive Masse des Katalysators vor dessen Reduktion mit Wasserstoff
90 bis 99,8 Gew.-% Zirkoniumdioxid (ZrO₂),
0,1 bis 5,0 Gew.-% sauerstoffhaltige Verbindungen des Palladiums und
0,1 bis 5,0 Gew.-% sauerstoffhaltige Verbindungen des Platins
enthält.

Erfindungsgemäß lassen sich Anilin und substituierte aromatische Amine, insbesondere substituierte Aniline, ohne die beschriebenen Nachteile herstellen, wenn Cyclohexanol oder entsprechend substituiertes Cyclohexanol mit Ammoniak und Wasserstoff bei erhöhter Temperatur an dem bimetallischen Zirkoniumdioxid-Katalysator umgesetzt werden, welcher Platin und Palladium enthält.

Für die Synthese in der Gasphase wird der Edukt-Alkohol gezielt, bevorzugt in einem Kreisgasstrom, verdampft und gasförmig dem Reaktor zugeführt. Das Kreisgas dient zum einen der Verdampfung des Edukt-Alkohols und zum anderen als Reaktionspartner für die Aminierung.

In der Kreisgasfahrweise werden die Ausgangsstoffe (Alkohol, Wasserstoff und Ammoniak) in einem Kreisgasstrom verdampft und gasförmig dem Reaktor zugeführt. Die Edukte (Alkohol und Ammoniak) können auch als wässrige Lösungen verdampft und mit dem Kreisgasstrom auf das Katalysatorbett geleitet werden.

Bevorzugte Reaktoren sind Rohrreaktoren. Beispiele für geeignete Reaktoren mit Kreisgasstrom finden sich in Ullmann's Encyclopedia of Industrial Chemistry, 5th Ed., Vol. B 4, Seiten 199-238, "Fixed-Bed Reactors".

Alternativ erfolgt die Umsetzung vorteilhaft in einem Rohrbündelreaktor oder in einer Monostranganlage.

Bei einer Monostranganlage kann der Rohrreaktor, in dem die Umsetzung erfolgt, aus einer Hintereinanderschaltung mehrerer (z.B. zweier oder dreier) einzelner Rohrreaktoren bestehen. Optional ist hier vorteilhaft eine Zwischeneinspeisung von Feed (enthaltend das Edukt und/oder Ammoniak und/oder H₂) und/oder Kreisgas und/oder Reaktoraustrag aus einem nachgeschalteten Reaktor möglich.

Bevorzugt findet keine Hintereinanderschaltung von zwei oder mehr Reaktoren statt.

Die Kreisgasmenge liegt bevorzugt im Bereich von 40 bis 1500 m³ (bei Betriebsdruck) / [m³ Katalysator (Schüttvolumen) h], insbesondere im Bereich von 100 bis 700 m³ (bei Betriebsdruck) / [m³ Katalysator (Schüttvolumen) · h].

Das Kreisgas enthält bevorzugt mindestens 10, besonders 50 bis 100, ganz besonders 80 bis 100, Vol.% H₂.

Für die Synthese in der Flüssigphase sind alle Edukte und Produkte geeignet, welche schwer verdampfbar oder thermisch labil sind. In diesen Fällen kommt als weiterer Vorteil hinzu, dass auf eine Verdampfung und Rekondensation des Amins im Prozess verzichtet werden kann.

Im erfindungsgemäßen Verfahren werden die Katalysatoren bevorzugt in Form von Katalysatoren eingesetzt, die nur aus katalytisch aktiver Masse und gegebenenfalls einem Verformungshilfsmittel (wie z.B. Graphit oder Stearinsäure), falls der Katalysator als Formkörper eingesetzt wird, bestehen, also keine weiteren katalytisch aktiven Begleitstoffe enthalten.
In diesem Zusammenhang wird das oxidische Trägermaterial Zirkoniumdioxid (ZrO₂) als zur katalytisch aktiven Masse gehörig gewertet.

Die Katalysatoren werden dergestalt eingesetzt, dass man die katalytisch aktive, zu Pulver vermahlene Masse in das Reaktionsgefäß einbringt oder, dass man die katalytisch aktive Masse nach Mahlung, Vermischung mit Formhilfsmitteln, Formung und Temperung als Katalysatorformkörper — beispielsweise als Tabletten, Kugeln, Ringe, Extrudate (z.B. Stränge) — im Reaktor anordnet.

Die Konzentrationsangaben (in Gew.-%) der Komponenten des Katalysators beziehen sich jeweils — falls nicht anders angegeben — auf die katalytisch aktive Masse des fertigen Katalysators nach dessen letzter Wärmebehandlung und vor dessen Reduktion mit Wasserstoff.

Die katalytisch aktive Masse des Katalysators, nach dessen letzter Wärmebehandlung und vor dessen Reduktion mit Wasserstoff, ist als die Summe der Massen der katalytisch aktiven Bestandteile und der o.g. Katalysatorträgermaterialien definiert und enthält im wesentlichen die folgenden Bestandteile:
Zirkoniumdioxid (ZrO₂), sauerstoffhaltige Verbindungen des Palladiums und sauerstoffhaltige Verbindungen des Platins.

Die Summe der o.g. Bestandteile der katalytisch aktiven Masse beträgt üblicherweise 70 bis 100 Gew.-%, bevorzugt 80 bis 100 Gew.-%, besonders bevorzugt 90 bis 100 Gew.-%, besonders > 95 Gew.-%, ganz besonders > 98 Gew.-%, insbesondere > 99 Gew.-%, z.B. besonders bevorzugt 100 Gew.-%.

Die katalytisch aktive Masse der im erfindungsgemäßen Verfahren eingesetzten Katalysatoren kann weiterhin ein oder mehrere Elemente (Oxidationsstufe 0) oder deren anorganische oder organische Verbindungen, ausgewählt aus den Gruppen I A bis VI A und I B bis VII B und VIII des Periodensystems der Elemente, enthalten.

Beispiele für solche Elemente bzw. deren Verbindungen sind:
Übergangsmetalle, wie Co bzw. CoO, Re bzw. Rheniumoxide, Mn bzw. MnO₂, Mo bzw. Molybdänoxide, W bzw. Wolframoxide, Ta bzw. Tantaloxide, Nb bzw. Nioboxide oder Nioboxalat, V bzw. Vanadiumoxide bzw. Vanadylpyrophosphat; Lanthanide, wie Ce bzw. CeO₂ oder Pr bzw. Pr₂O₃; Alkalimetalloxide, wie Na₂O; Alkalimetallcarbonate; Erdalkalimetalloxide, wie SrO; Erdalkalimetallcarbonate, wie MgCO₃, CaCO₃ und Ba-CO₃; Boroxid (B₂O₃).

Bevorzugt enthält die katalytisch aktive Masse der im erfindungsgemäßen Verfahren eingesetzten Katalysatoren kein Ruthenium, kein Kupfer, kein Cadmium, kein Zink, kein Cobalt, kein Eisen und/oder kein Nickel.

Die katalytisch aktive Masse der im erfindungsgemäßen Verfahren eingesetzten Katalysatoren enthält nach deren letzter Wärmebehandlung und vor dessen Reduktion mit Wasserstoff
90 bis 99,8 Gew.-%, bevorzugt 98 bis 99,6 Gew.-%, besonders bevorzugt 98,8 bis 99,2 Gew.-% Zirkoniumdioxid (ZrO₂),
0,1 bis 5,0 Gew.-%, bevorzugt 0,2 bis 1,0 Gew.-%, besonders bevorzugt 0,4 bis
0,6 Gew.-%, sauerstoffhaltige Verbindungen des Palladiums und
0,1 bis 5,0 Gew.-%, bevorzugt 0,2 bis 1,0 Gew.-%, besonders bevorzugt 0,4 bis
0,6 Gew.-%, sauerstoffhaltige Verbindungen des Platins.

Zur Herstellung der im erfindungsgemäßen Verfahren verwendeten Katalysatoren sind verschiedene Verfahren möglich. Hier sind z.B. die bekannten Fällungsmethoden zu nennen.

Die im erfindungsgemäßen Verfahren verwendeten Katalysatoren können insbesondere durch Tränkung von Zirkoniumdioxid (ZrO₂) das beispielsweise in Form von Pulver oder Formkörpern, wie Strängen, Tabletten, Kugeln oder Ringen, vorliegt, hergestellt werden.

Das Zirkoniumdioxid wird beispielsweise in der monoklinen oder tetragonalen Form, bevorzugt in der monoklinen Form eingesetzt.

Die Herstellung von Formkörpern kann nach den üblichen Verfahren erfolgen.

Die Tränkung erfolgt ebenfalls nach den üblichen Verfahren, wie z.B. in A. B. Stiles, Catalyst Manufacture -Laboratory and Commercial Preparations, Marcel Dekker, New York (1983) beschrieben, durch Aufbringung einer jeweils entsprechenden Metallsalzlösung in einer oder mehreren Tränkstufen, wobei als Metallsalze z.B. entsprechende Nitrate, Acetate oder Chloride verwendet werden. Die Masse wird im Anschluss an die Tränkung getrocknet und optional kalziniert.

Die Tränkung kann nach der sogenannten "incipient wetness"-Methode erfolgen, bei der das Zirkoniumdioxid entsprechend seiner Wasseraufnahmekapazität maximal bis zur Sättigung mit der Tränklösung befeuchtet wird. Die Tränkung kann aber auch in überstehender Lösung erfolgen.

Bei mehrstufigen Tränkverfahren ist es zweckmäßig, zwischen einzelnen Tränkschritten zu trocknen und optional zu kalzinieren. Die mehrstufige Tränkung ist vorteilhaft besonders dann anzuwenden, wenn das Zirkoniumdioxid mit einer größeren Metallmenge beaufschlagt werden soll.

Zur Aufbringung der Metallkomponenten auf das Zirkoniumdioxid kann die Tränkung gleichzeitig mit allen Metallsalzen oder in beliebiger Reihenfolge der einzelnen Metallsalze nacheinander erfolgen.

Nach der Calcinierung, die z.B. bei einer Temperatur im Bereich von 200 bis 600°C durchgeführt wird, wird der Katalysator zweckmäßigerweise konditioniert, sei es, dass man ihn durch Vermahlen auf eine bestimmte Korngröße einstellt oder dass man ihn nach seiner Vermahlung mit Formhilfsmitteln wie Graphit oder Stearinsäure vermischt, mittels einer Presse zu Formlingen, z.B. Tabletten, verpresst und tempert. Die Tempertemperaturen entsprechen dabei bevorzugt den Temperaturen bei der Calcinierung.

Die auf diese Weise hergestellten Katalysatoren enthalten die katalytisch aktiven Metalle in Form eines Gemisches ihrer sauerstoffhaltigen Verbindungen, d.h. insbesondere als Oxide und Mischoxide.

Die auf diese Weise hergestellten Katalysatoren werden als solche gelagert und ggf. gehandelt. Vor ihrem Einsatz als Katalysatoren werden sie üblicherweise vorreduziert. Sie können jedoch auch ohne Vorreduktion eingesetzt werden, wobei sie dann unter den Bedingungen der hydrierenden/dehydrierenden Aminierung durch den im Reaktor vorhandenen Wasserstoff reduziert werden.

Zur Vorreduktion werden die Katalysatoren zunächst bei bevorzugt 150 bis 200°C über einen Zeitraum von z.B. 12 bis 20 Stunden einer Stickstoff-Wasserstoff-Atmosphäre ausgesetzt und anschließend noch bis zu ca. 24 Stunden bei bevorzugt 200 bis 400°C in einer Wasserstoffatmosphäre behandelt. Bei dieser Vorreduktion wird ein Teil der in den Katalysatoren vorliegenden sauerstoffhaltigen Metallverbindungen zu den entsprechenden Metallen reduziert, so dass diese gemeinsam mit den verschiedenartigen Sauerstoffverbindungen in der aktiven Form des Katalysators vorliegen.

Ausgewählte, in EP-A-701 995 (BASF AG) offenbarte, Pd/Pt/ZrO₂-Katalysatoren werden im erfindungsgemäßen Verfahren besonders bevorzugt eingesetzt.

Das erfindungsgemäße Verfahren wird kontinuierlich durchgeführt, wobei der Katalysator bevorzugt als Festbett im Reaktor angeordnet ist. Dabei ist sowohl eine Anströmung des Katalysatorfestbetts von oben als auch von unten möglich. Der Gasstrom wird dabei durch Temperatur, Druck und Menge so eingestellt, dass auch schwerer siedende (hoch siedende) Reaktionsprodukte in der Gasphase verbleiben.

Das Aminierungsmittel Ammoniak kann bezüglich der zu aminierenden alkoholischen Hydroxylgruppe in stöchiometrischen, unter- oder überstöchiometrischen Mengen eingesetzt werden.

Ammoniak wird im allgemeinen mit einem 1,5 bis 250-fachen, bevorzugt 2 bis 100-fachen, insbesondere 2 bis 10-fachen molaren Überschuss pro Mol umzusetzender alkoholischer Hydroxylgruppe eingesetzt.
Höhere Überschüsse an Ammoniak sind möglich.

Bevorzugt wird eine Abgasmenge von 5 bis 800 Normkubikmeter/h, insbesondere 20 bis 300 Normkubikmeter/h, gefahren.

Beim Arbeiten in der Flüssigphase leitet man die Edukte (Alkohol plus Ammoniak) simultan in flüssiger Phase bei Drücken von im allgemeinen 5 bis 30 MPa (50-300 bar), bevorzugt 5 bis 25 MPa, besonders bevorzugt 15 bis 25 MPa, und Temperaturen von im allgemeinen 80 bis 350°C, besonders 100 bis 300°C, bevorzugt 120 bis 270°C, besonders bevorzugt 130 bis 250°C, insbesondere 170 bis 230°C, inklusive Wasserstoff über den Katalysator, der sich üblicherweise in einem bevorzugt von außen beheizten Festbettreaktor befindet. Es ist dabei sowohl eine Rieselfahrweise als auch eine Sumpffahrweise möglich. Die Katalysatorbelastung liegt im allgemeinen im Bereich von 0,05 bis 5, bevorzugt 0,1 bis 2, besonders bevorzugt 0,2 bis 0,6, kg Alkohol pro Liter Katalysator (Schüttvolumen) und Stunde. Gegebenenfalls kann eine Verdünnung der Edukte mit einem geeigneten Lösungsmittel, wie Tetrahydrofuran, Dioxan, N-Methylpyrrolidon oder Ethylenglykoldimethylether, erfolgen. Es ist zweckmäßig, die Reaktanden bereits vor der Zuführung in das Reaktionsgefäß zu erwärmen, und zwar bevorzugt auf die Reaktionstemperatur.

Beim Arbeiten in der Gasphase werden die gasförmigen Edukte (Alkohol plus Ammoniak) in einem zur Verdampfung ausreichend groß gewählten Gasstrom, bevorzugt Wasserstoff, bei Drücken von im allgemeinen 0,1 bis 40 MPa (1 bis 400 bar), bevorzugt 0,1 bis 10 MPa, besonders bevorzugt 0,1 bis 5 MPa, in Gegenwart von Wasserstoff über den Katalysator geleitet. Die Temperaturen für die Aminierung betragen im allgemeinen 80 bis 350°C, besonders 100 bis 300°C, bevorzugt 120 bis 290°C, besonders bevorzugt 160 bis 280°C. Es ist dabei sowohl eine Anströmung des Katalysatorfestbetts von oben als auch von unten möglich. Den erforderlichen Gasstrom erhält man bevorzugt durch eine Kreisgasfahrweise.
Die Katalysatorbelastung liegt im allgemeinen im Bereich von 0,01 bis 2, bevorzugt 0,05 bis 0,5, kg Alkohol pro Liter Katalysator (Schüttvolumen) und Stunde.

Der Wasserstoff wird der Reaktion im allgemeinen in einer Menge von 5 bis 400 I, bevorzugt in einer Menge von 50 bis 200 I pro Mol Alkoholkomponente zugeführt, wobei die Literangaben jeweils auf Normalbedingungen umgerechnet wurden (S.T.P.).

Sowohl beim Arbeiten in der Flüssigphase als auch beim Arbeiten in der Gasphase ist die Anwendung höherer Temperaturen und höherer Gesamtdrücke und Katalysatorbelastungen möglich. Der Druck im Reaktionsgefäß, welcher sich aus der Summe der Partialdrücke des Aminierungsmittels Ammoniak, des Alkohols und der gebildeten Reaktionsprodukte sowie ggf. des mitverwendeten Lösungsmittels bei den angegebenen Temperaturen ergibt, wird zweckmäßigerweise durch Aufpressen von Wasserstoff auf den gewünschten Reaktionsdruck erhöht.

Sowohl beim kontinuierlichen Arbeiten in der Flüssigphase als auch beim kontinuierlichen Arbeiten in der Gasphase kann das überschüssige Aminierungsmittel zusammen mit dem Wasserstoff im Kreis geführt werden.

Ist der Katalysator als Festbett angeordnet, kann es für die Selektivität der Reaktion vorteilhaft sein, die Katalysatorformkörper im Reaktor mit inerten Füllkörpern zu vermischen, sie sozusagen zu "verdünnen". Der Anteil der Füllkörper in solchen Katalysatorzubereitungen kann 20 bis 80, besonders 30 bis 60 und insbesonders 40 bis 50 Volumenteile betragen.

Das im Zuge der Umsetzung gebildete Reaktionswasser (jeweils ein Mol pro Mol umgesetzte Alkoholgruppe) wirkt sich im allgemeinen auf den Umsetzungsgrad, die Reaktionsgeschwindigkeit, die Selektivität und die Katalysatorstandzeit nicht störend aus und wird deshalb zweckmäßigerweise erst bei der Aufarbeitung des Reaktionsproduktes aus diesem entfernt, z.B. destillativ.

Aus dem Reaktionsaustrag werden, nachdem dieser zweckmäßigerweise entspannt worden ist, der überschüssige Wasserstoff und das gegebenenfalls vorhandene überschüssige Aminierungsmittel entfernt und das erhaltene Reaktionsrohprodukt gereinigt, z.B. durch eine fraktionierende Rektifikation. Das überschüssige Aminierungsmittel und der Wasserstoff werden vorteilhaft wieder in die Reaktionszone zurückgeführt. Das gleiche gilt für die eventuell nicht vollständig umgesetzte Alkoholkomponente.

Die jeweiligen Reinprodukte können aus den Rohwaren durch Rektifikation nach den bekannten Methoden erhalten werden. Die Reinprodukte fallen als Azeotrope mit Wasser an oder können in Anlehnung an die Patentanmeldungen EP-A-1 312 599 und EP-A-1 312 600 (beide BASF AG) durch eine Flüssig-flüssig-Extraktion mit konzentrierter Natronlauge entwässert werden. Diese Entwässerung kann vor oder nach der Reindestillation erfolgen. Eine destillative Entwässerung in Gegenwart eines Schleppmittels nach bekannten Methoden ist auch möglich.

Für den Fall, dass die Rohware oder das aromatische Amin in der Rohware kaum oder nicht mit Wasser mischbar sind, ist eine Entwässerung durch eine Trennung der organischen und der wässrigen Phase mit bekannten Methoden auch möglich. Gemäß der gelehrten Vorgehensweise in EP-A-1 312 599 und in EP-A-1 312 600 (beide BASF AG) können von der separierten organischen Phase in einem Schritt eine oder mehrere Leichtsiederfraktionen von dem aminhaltigen Gemisch destillativ abgetrennt werden. In einem weiteren Schritt besteht die Möglichkeit, eine oder mehrere Schwersiederfraktionen von dem aminhaltigen Gemisch destillativ abzutrennen. In einem folgenden Destillationsschritt kann aus der Mischung das im wesentlichen wasserfreie Amin als Sumpfabzug oder Seitenabzug der Kolonne in reiner Form erhalten werden, welches gegebenenfalls einer weiteren Aufreinigung oder Auftrennung unterworfen wird.

Diese Einzelschritte zur Aufreinigung des Amins können gegebenenfalls auch in einer einzigen Kolonne batchweise oder kontinuierlich durchgeführt werden, wobei eine Abtrennung der Leichtsieder über den Kopfabzug und/oder den Seitenabzug des Verstärkungsteils der Kolonne, eine Abtrennung der Schwersiederfraktionen über den Sumpfabzug der Destillationskolonne und die Abtrennung des reinen Amins über den Seitenabzug im Abtriebsteil der Kolonne erfolgen kann.

In einer besonders bevorzugten Variante kommt als kontinuierliche Destillationskolonne eine Trennwandkolonne zum Einsatz.

Unumgesetzte Edukte und gegebenenfalls anfallende geeignete Nebenprodukte können wieder in die Synthese zurückgeführt werden. Nicht umgesetzte Edukte können in diskontinuierlicher oder kontinuierlicher Fahrweise nach Kondensation der Produkte im Abscheider in dem Kreisgasstrom erneut über das Katalysatorbett geströmt werden.

Durch die Verwendung von Ammoniak als Aminierungsmittel wird die alkoholische Hydroxylgruppe des eingesetzten cycloaliphatischen Alkohols unter Erhalt der Position am aliphatischen Ring in die primäre Aminogruppe (-NH₂) umgewandelt.

Als cycloaliphatische Alkohole eignen sich praktisch alle Alkohole mit aliphatischer OH-Funktion. D.h. die OH-Gruppe ist an ein sp³-hybridisiertes C-Atom in einem aliphatischen Ring gebunden. Der aliphatische Ring kann neben C-Atomen auch ein oder mehrere Heteroatome, wie N, O oder S, aufweisen. Die Alkohole können ferner Substituenten tragen oder funktionelle Gruppen enthalten, welche sich unter den Bedingungen der hydrierenden/dehydrierenden Aminierung inert verhalten, beispielsweise Alkoxy-, Alkenyloxy-, Alkylamino- oder Dialkylaminogruppen, oder auch gegebenenfalls unter den Bedingungen der hydrierenden Aminierung hydriert werden, beispielsweise CC-Doppel- oder Dreifachbindungen. Sollen mehrwertige cycloaliphatische Alkohole aminiert werden, so hat man es über die Steuerung der Reaktionsbedingungen in der Hand, bevorzugt entsprechende Aminoalkohole oder mehrfach aminierte Produkte zu erhalten.

Bevorzugt werden beispielsweise die folgenden cycloaliphatischen Alkohole aminiert: Cyclohexanol, wobei der Cyclohexylrest einen oder mehrere Alkylreste, insbesondere C₁₋₉-Alkylreste und C₅₋₆-Cycloalkylreste, und/oder Arylreste als Substituenten tragen kann.

C₁₋₉-Alkylreste, bevorzugt C₁₋₃-Alkylreste, sind z.B. Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, Cyclopentylmethyl, n-Heptyl, iso-Heptyl, Cyclohexylmethyl, n-Octyl, iso-Octyl, 2-Ethylhexyl, n-Nonyl.

Arylreste sind z.B. Phenyl, 1-Naphthyl, 2-Naphthyl.

Weitere Beispiele für bevorzugt eingesetzte cycloaliphatischen Alkohole sind: 2,6-Dimethylcyclohexanol, 2,4-Dimethylcyclohexanol, 3,5-Dimethylcyclohexanol, 2,3,6-Trimethylcyclohexanol, 2,4,6-Trimethylcyclohexanol, 2,6-Diethylcyclohexanol, 2-Methyl-6-ethylcyclohexanol, 2,6-Diisopropylcyclohexanol, 2,6-Di-sec.-Butylcyclohexanol, 3-tert.-Butylcyclohexanol, 2-Isopropyl-6-methylcyclohexanol und 2-Isopropyl-6-ethylcyclohexanol.

Die als Edukt eingesetzten cycloaliphatischen Alkohole, insbesondere Cyclohexanole, sind gut zugängliche Verbindungen.

Die als Edukt eingesetzten cycloaliphatischen Alkohole, insbesondere Cyclohexanole, ganz besonders Cyclohexanol und 2,6-Dimethylcyclohexanol, weisen bevorzugt eine Reinheit von ≥ 95 Gew.-%, besonders ≥ 98 Gew.-%, auf.
Der Gehalt an Ketonen, insbesondere an Cyclohexanonen, beträgt bevorzugt ≤ 2 Gew.-%, besonders ≤ 1 Gew.-%, und liegt ganz besonders im Bereich von 0 bis ≤ 0,5 Gew.-%, weiter besonders im Bereich von 0 bis ≤ 0,2 Gew.-%.

Beispielsweise kann als, Edukt kommerziell erhältliches 2,6-Dimethylcyclohexanol verwendet werden, das einen Gehalt von mindestens 70 Gew.-% (z.B. von Aldrich, ABCR, ASDI-Inter, ICN-RF, VWR), bevorzugt mindestens 95 Gew.-% (z.B. von TCI-JP, TCI-US), besonders bevorzugt mindestens 99 Gew.-% aufweist. Das besonders bevorzugt eingesetzte Edukt 2,6-Dimethylcyclohexanol mit ≥ 99 Gew.-% Reinheit haben verschiedene Lieferanten in ihrem Sortiment, beispielhaft seien die Firmen Acros und Kanto genannt. 98 Gew.-%ige Ware erhält man beispielsweise bei Pfaltz-Bauer oder Wiley sowie anderen Anbietern.

Mit dem erfindungsgemäßen Verfahren bevorzugt hergestellte aromatische Amine, insbesondere substituierte Aniline, sind 2,6-Di-(C₁₋₉-alkyl)-aniline aus den entsprechenden 2,6-Di-(C₁₋₉-alkyl)-cyclohexanolen. Beispiele sind 2,6-Dimethylanilin (2,6-Xylidin), 3,5-Dimethylanilin, 2,6-Diethylanilin, 2-Methyl-6-ethylanilin, 2,6-Diisopropylanilin, 2-Isopropyl-6-methylanilin und 2-Isopropyl-6-ethylanilin.

Mit dem erfindungsgemäßen Verfahren besonders bevorzugt hergestelltes aromatisches Amin ist 2,6-Dimethylanilin (2,6-Xylidin) durch Umsetzung von 2,6-Dimethylcyclohexanol.

Mit dem erfindungsgemäßen Verfahren, insbesondere gemäß den Ansprüchen 17 oder 18, ist insbesondere 2,6-Dimethylanilin (2,6-Xylidin) mit einer Reinheit von ≤ 99 Gew.-%, besonders ≥ 99,5 Gew.-%, ganz besonders ≥ 99,85 Gew.-%, und einem Gehalt an 2,6-Dimethylphenol von ≤ 0,1 Gew.-%, besonders ≤ 0,05 Gew.-%, ganz besonders ≤ 0,02 Gew.-%, z.B. 0 bis 0,015 Gew.-%, aus 2,6-Dimethylcyclohexanol und Ammoniak herstellbar.

Die o.g. Gehalte in Gew.-% werden wie folgt gaschromatografisch bestimmt:

| | | |
|---|---|---|
| Trennsäule | : | DB WAX ( Polyethylenglycol) |
| Länge (m) | : | 30 |
| Filmdicke (µm) | | 0,5 |
| Innendurchmesser (mm) | | 0,25 |
| Trägergas | : | Helium |
| Vordruck (bar) | | 1,0 |
| Split (ml/Min.) | : | 100 |
| Septumspülung (ml/Min.) | | 4 |
| Ofentemperatur (°C) | : | 80 |
| Vorheizzeit (Min.) | | 3 |
| Rate (°C/Min.) | | 5 |
| Ofentemperatur (°C) | | 240 |
| Nachheizzeit (Min.) | : | 30 |
| Injektortemperatur (°C) | | 250 |
| Detektortemperatur (°C) | | 260 |
| Injektion | | HP 7673-Autosampler |
| Injetionsvolumen (mikrol) | | 0,2 |
| Detektortyp | : | FID |

### Beispiele

Für alle Beispiele wurde der ausgewählte bimetallische Palladium-/Platin-Katalysator gemäß Beispiel 4 (Seite 6, Zeilen 12-15) von EP-B1-701 995 verwendet und auch nach der dort beschriebenen Methode (Seite 4, Zeilen 47-52) aktiviert. Danach wurde der geträgerte Edelmetall-Katalysator in den Reaktor eingebaut und anschließend in einem Stickstoff-/Wasserstoffstrom drucklos oder unter Betriebsdruck bei 200°C reduziert.

### Herstellung von 2,6-Dimethylanilin

In einem Rohr-Reaktor mit 10 Liter Katalysatorfüllung wurde bei 2 bar Gesamtdruck ein Kreisgasstrom aus 170 kg/h Ammoniak und 20 kg/h Wasserstoff eingestellt. In diesen Strom wurden kontinuierlich 122 kg/h 2,6-Dimethylcyclohexanol gegeben und verdampft. Die gasförmige Mischung wurde bei 200 bis 270°C über das Katalysatorbett geströmt. Die Ausbeute an 2,6-Xylidin nach dem Reaktor betrug größer 90 %.

## Patentansprüche

1. Verfahren zur kontinuierlichen Herstellung eines primären aromatischen Amins durch Umsetzung eines entsprechenden cycloaliphatischen Alkohols mit Ammoniak in Gegenwart von Wasserstoff bei einer Temperatur im Bereich von 80 bis 350°C in Gegenwart eines Heterogenkatalysators, **dadurch gekennzeichnet, dass** die katalytisch aktive Masse des Katalysators vor dessen Reduktion mit Wasserstoff
90 bis 99,8 Gew.-% Zirkoniumdioxid (ZrO₂)
0,1 bis 5,0 Gew.-% sauerstoffhaltige Verbindungen des Palladiums und
0,1 bis 5,0 Gew.-% sauerstoffhaltige Verbindungen des Platins enthält.

2. Verfahren nach dem vorhergehenden Anspruch, **dadurch gekennzeichnet, dass** man die Umsetzung bei einer Temperatur im Bereich von 120 bis 300°C durchführt.

3. Verfahren nach einem der vorhergehenden Ansprüche, **dadurch gekennzeichnet, dass** man die Umsetzung in der Flüssigphase bei einem Absolutdruck im Bereich von 5 bis 30 MPa oder in der Gasphase bei einem Absolutdruck im Bereich von 0,1 bis 40 MPa durchführt.

4. Verfahren nach einem der vorhergehenden Ansprüche, **dadurch gekennzeichnet, dass** die katalytisch aktive Masse des Katalysators vor dessen Reduktion mit Wasserstoff
98 bis 99,6 Gew.-% Zirkoniumdioxid (ZrO₂),
0,2 bis 1,0 Gew.-% sauerstoffhaltige Verbindungen des Palladiums und
0,2 bis 1,0 Gew.-% sauerstoffhaltige Verbindungen des Platins enthält.

5. Verfahren nach einem der Ansprüche 1 bis 3, **dadurch gekennzeichnet, dass** die katalytisch aktive Masse des Katalysators vor dessen Reduktion mit Wasserstoff
98,8 bis 99,2 Gew.-% Zirkoniumdioxid (ZrO₂)
0,4 bis 0,6 Gew.-% sauerstoffhaltige Verbindungen des Palladiums und
0,4 bis 0,6 Gew.-% sauerstoffhaltige Verbindungen des Platins enthält.

6. Verfahren nach einem der vorhergehenden Ansprüche, **dadurch gekennzeichnet, dass** der Ammoniak in der 1,5- bis 250-fachen molaren Menge bezogen auf den eingesetzten cycloaliphatischen Alkohol eingesetzt wird.

7. Verfahren nach einem der Ansprüche 1 bis 5, **dadurch gekennzeichnet, dass** der Ammoniak in der 2,0- bis 10-fachen molaren Menge bezogen auf den eingesetzten cycloaliphatischen Alkohol eingesetzt wird.

8. Verfahren nach einem der vorhergehenden Ansprüche, **dadurch gekennzeichnet, dass** der Katalysator im Reaktor als Festbett angeordnet ist.

9. Verfahren nach einem der vorhergehenden Ansprüche, **dadurch gekennzeichnet, dass** die Umsetzung in einem Rohrreaktor erfolgt.

10. Verfahren nach einem der vorhergehenden Ansprüche, **dadurch gekennzeichnet, dass** die Umsetzung bei einer Kreisgasfahrweise erfolgt.

11. Verfahren nach einem der vorhergehenden Ansprüche, **dadurch gekennzeichnet, dass** man den Alkohol als wässrige Lösung einsetzt.

12. Verfahren nach einem der vorhergehenden Ansprüche, **dadurch gekennzeichnet, dass** man den Ammoniak als wässrige Lösung einsetzt.

13. Verfahren nach einem der vorhergehenden Ansprüche zur Herstellung eines Phenylamins, wobei der Phenylrest einen oder mehrere C₁₋₉-Alkylreste als Substituenten tragen kann.

14. Verfahren nach einem der Ansprüche 1 bis 12 zur Herstellung von Anilin durch Umsetzung von Cyclohexanol mit Ammoniak.

15. Verfahren nach einem der Ansprüche 1 bis 12 zur Herstellung eines 2,6-Di-(C₁₋₉-alkyl)-anilins.

16. Verfahren nach einem der Ansprüche 1 bis 12 zur Herstellung von 2,6-Dimethylanilin (2,6-Xylidin) durch Umsetzung von 2,6-Dimethylcyclohexanol mit Ammoniak.

17. Verfahren nach einem der vorhergehenden Ansprüche, **dadurch gekennzeichnet, dass** man aus dem Reaktionsrohprodukt die organische Phase abtrennt und diese nachfolgend in einer Destillationskolonne kontinuierlich destillativ auftrennt, wobei das primäre aromatische Amin über einen seitlichen Abzug im Abtriebsteil der Kolonne, Leichtersieder und Wasser über Kopf und Schwersieder über Sumpf abgezogen werden.

18. Verfahren nach dem vorhergehenden Anspruch, **dadurch gekennzeichnet, dass** es sich bei der Destillationskolonne um eine Trennwandkolonne handelt.

## Claims

1. A process for continuously preparing a primary aromatic amine by reacting a corresponding cycloaliphatic alcohol with ammonia in the presence of hydrogen at a temperature in the range from 80 to 350°C in the presence of a heterogeneous catalyst, wherein the catalytically active composition of the catalyst, before its reduction with hydrogen, comprises
from 90 to 99.8% by weight of zirconium dioxide (ZrO₂),
from 0.1 to 5.0% by weight of oxygen compounds of palladium and
from 0.1 to 5.0% by weight of oxygen compounds of platinum.

2. The process according to the preceding claim, wherein the reaction is carried out at a temperature in the range from 120 to 300°C.

3. The process according to either of the preceding claims, wherein the reaction is carried out in the liquid phase at an absolute pressure in the range from 5 to 30 MPa or in the gas phase at an absolute pressure in the range from 0.1 to 40 MPa.

4. The process according to any of the preceding claims, wherein the catalytically active composition of the catalyst, before its reduction with hydrogen, comprises
from 98 to 99.6% by weight of zirconium dioxide (ZrO₂),
from 0.2 to 1.0% by weight of oxygen compounds of palladium and
from 0.2 to 1.0% by weight of oxygen compounds of platinum.

5. The process according to any of claims 1 to 3, wherein the catalytically active composition of the catalyst, before its reduction with hydrogen, comprises
from 98.8 to 99.2% by weight of zirconium dioxide (ZrO₂),
from 0.4 to 0.6% by weight of oxygen compounds of palladium and
from 0.4 to 0.6% by weight of oxygen compounds of platinum.

6. The process according to any of the preceding claims, wherein the ammonia is used in from 1.5 to 250 times the molar amount of the cycloaliphatic alcohol used.

7. The process according to any of claims 1 to 5, wherein the ammonia is used in from 2.0 to 10 times the molar amount of the cycloaliphatic alcohol used.

8. The process according to any of the preceding claims, wherein the catalyst is arranged in the reactor as a fixed bed.

9. The process according to any of the preceding claims, wherein the reaction is effected in a tubular reactor.

10. The process according to any of the preceding claims, wherein the reaction is effected in a cycle gas method.

11. The process according to any of the preceding claims, wherein the alcohol is used as an aqueous solution.

12. The process according to any of the preceding claims, wherein the ammonia is used as an aqueous solution.

13. The process according to any of the preceding claims for preparing a phenylamine, where the phenyl radical may bear one or more C₁₋₉-alkyl radicals as substituents.

14. The process according to any of claims 1 to 12 for preparing aniline by reacting cyclohexanol with ammonia.

15. The process according to any of claims 1 to 12 for preparing a 2,6-di(C₁₋₉-alkyl) aniline.

16. The process according to any of claims 1 to 12 for preparing 2,6-dimethylaniline (2,6-xylidine) by reacting 2,6-dimethylcyclohexanol with ammonia.

17. The process according to any of the preceding claims, wherein the organic phase is removed from the crude reaction product and is subsequently separated by distillation continuously in a distillation column, the primary aromatic amine being drawn off via a side draw in the stripping section of the column, low boilers and water via the top, and high boilers via the bottom.

18. The process according to the preceding claim, wherein the distillation column is a dividing wall column.

## Revendications

1. Procédé pour la préparation continue d'une amine aromatique primaire par transformation d'un alcool cycloaliphatique correspondant avec de l'ammoniac en présence d'hydrogène à une température dans la plage de 80 à 350°C en présence d'un catalyseur hétérogène, **caractérisé en ce que** la masse catalytiquement active du catalyseur contient, avant sa réduction avec l'hydrogène
90 à 99,8% en poids de dioxyde de zirconium (ZrO₂),
0,1 à 5,0% en poids de composés oxygénés du palladium et
0,1 à 5,0% en poids de composés oxygénés du platine.

2. Procédé selon la revendication précédente, **caractérisé en ce qu'**on réalise la transformation à une température dans la plage de 120 à 300°C.

3. Procédé selon l'une quelconque des revendications précédentes, **caractérisé en ce qu'**on réalise la transformation en phase liquide à une pression absolue dans la plage de 5 à 30 MPa ou en phase gazeuse une pression absolue dans la plage de 0,1 à 40 MPa.

4. Procédé selon l'une quelconque des revendications précédentes, **caractérisé en ce que** la masse catalytiquement active du catalyseur contient, avant sa réduction avec l'hydrogène
98 à 99,6% en poids de dioxyde de zirconium (ZrO₂),
0,2 à 1,0% en poids de composés oxygénés du palladium et
0,2 à 1,0% en poids de composés oxygénés du platine.

5. Procédé selon l'une quelconque des revendications 1 à 3, **caractérisé en ce que** la masse catalytiquement active du catalyseur contient, avant sa réduction avec l'hydrogène
98,8 à 99,2% en poids de dioxyde de zirconium (ZrO₂),
0,4 à 0,6% en poids de composés oxygénés du palladium et
0,4 à 0,6% en poids de composés oxygénés du platine.

6. Procédé selon l'une quelconque des revendications précédentes, **caractérisé en ce que** l'ammoniac est utilisé en une quantité représentant 1,5 à 250 fois la quantité molaire par rapport à l'alcool cycloaliphatique utilisé.

7. Procédé selon l'une quelconque des revendications 1 à 5, **caractérisé en ce que** l'ammoniac est utilisé en une quantité représentant 2,0 à 10 fois la quantité molaire par rapport à l'alcool cycloaliphatique utilisé.

8. Procédé selon l'une quelconque des revendications précédentes, **caractérisé en ce que** le catalyseur est disposé dans le réacteur sous forme de lit fixe.

9. Procédé selon l'une quelconque des revendications précédentes, **caractérisé en ce que** la transformation a lieu dans un réacteur tubulaire.

10. Procédé selon l'une quelconque des revendications précédentes, **caractérisé en ce que** la transformation a lieu dans un mode opératoire avec un gaz en circulation.

11. Procédé selon l'une quelconque des revendications précédentes, **caractérisé en ce qu'**on utilise l'alcool sous forme de solution aqueuse.

12. Procédé selon l'une quelconque des revendications précédentes, **caractérisé en ce qu'**on utilise l'ammoniac sous forme de solution aqueuse.

13. Procédé selon l'une quelconque des revendications précédentes pour la préparation d'une phénylamine, où le radical phényle peut porter un ou plusieurs radicaux C₁₋₉-alkyle comme substituants.

14. Procédé selon l'une quelconque des revendications 1 à 12 pour la préparation d'aniline par transformation de cyclohexanol avec de l'ammoniac.

15. Procédé selon l'une quelconque des revendications 1 à 12 pour la préparation d'une 2,6-di-(C₁₋₉-alkyl)-aniline.

16. Procédé selon l'une quelconque des revendications 1 à 12 pour la préparation de 2,6-diméthylaniline (2,6-xylidine) par transformation de 2,6-diméthylcyclohexanol avec de l'ammoniac.

17. Procédé selon l'une quelconque des revendications précédentes, **caractérisé en ce qu'**on sépare la phase organique du produit de réaction brut et on la sépare ensuite par distillation continue dans une colonne de distillation, où l'amine aromatique primaire est soutirée via un soutirage latéral dans la partie d'épuisement de la colonne, les fractions à bas point d'ébullition et l'eau sont soutirées via la tête et les fractions à point d'ébullition élevé sont soutirées via le fond.

18. Procédé selon la revendication précédente, **caractérisé en ce qu'**il s'agit, pour la colonne de distillation, d'une colonne à paroi de séparation.
